Europäisches Patentamt

European Patent Office

Office européen des brevets

(19)

(11) Veröffentlichungsnummer: **0 012 415**
**A1**

(12) # EUROPÄISCHE PATENTANMELDUNG

(21) Anmeldenummer: 79105046.1

(22) Anmeldetag: 10.12.79

(51) Int. Cl.³: **G 01 N 27/46**
**// G01N31/06**

(30) Priorität: 16.12.78 DE 2854467

(43) Veröffentlichungstag der Anmeldung: **25.06.80**
**Patentblatt 80/13**

(84) Benannte Vertragsstaaten: **CH DE FR NL**

(71) Anmelder: **BAYER AG, Zentralbereich Patente, Marken und Lizenzen, D-5090 Leverkusen 1, Bayerwerk (DE)**

(72) Erfinder: **Harzdorf, Claus, Dr., Zum Hahnenberg 4, D-5068 Odenthal (DE)**
Erfinder: **Fritze, Ulrich, Dr., Haferkamp 6, D-5000 Köln 80 (DE)**
Erfinder: **Herschinger, Heinz, Zedernweg 1, D-5090 Leverkusen 31 (DE)**

(54) Verfahren und Vorrichtung zur kontinuierlichen Messung von Gasspuren.

(57) Das zu untersuchende Gas wird zusammen mit einer Absorptionsflüssigkeit kontinuierlich zerstäubt, so daß ein hoher Anteil des Gases in der Flüssigkeit absorbiert wird. Anschließend wird die flüssige Phase wieder abgetrennt und in kontinuierlicher Strömung durch eine elektrochemische Meßzelle mit einer für voltametrische Messungen geeignete Feststoffelektrode geleitet. Die Messung erfolgt zweckmäßig mit Hilfe einer potentiostatischen Meßschaltung.

EP 0 012 415 A1

BAYER AKTIENGESELLSCHAFT          5090 Leverkusen,Bayerwerk
                                  Ki/bc
Zentralbereich
Patente, Marken und Lizenzen

Verfahren und Vorrichtung zur kontinuierlichen Messung
von Gasspuren

Die Erfindung betrifft ein Verfahren zur Messung von Gasspuren, bei dem das zu untersuchende Gas zusammen mit einer Absorptionsflüssigkeit kontinuierlich zerstäubt, anschließend die flüssige Phase abgetrennt und in kontinuierlicher Strömung durch eine elektrochemische Meßzelle geleitet wird.

Ferner betrifft die Erfindung eine Vorrichtung zur Durchführung des Meßverfahrens.

Dieses Meßverfahren ist in der deutschen Patentanmeldung
P 27 23 310.9 ausführlich beschrieben. Die in der Flüssigkeit gebildeten Ionen werden hier mit Hilfe einer ionensensitiven Elektrode nachgewiesen und gemessen. Ein gewisser Nachteil der ionensensitiven Elektrode besteht
darin, daß manche in der Flüssigkeit löslichen Gaskompo-

Le A 19 351

nenten nicht gemessen werden können, da keine geeigneten ionensensitiven Elektroden zur Verfügung stehen.

Der Erfindung liegt daher die Aufgabe zugrunde, den Anwendungsbereich des oben beschriebenen Meßverfahrens zu erweitern.

Diese Aufgabe wird, ausgehend von dem eingangs beschriebenen Verfahren, erfindungsgemäß dadurch gelöst, daß statt einer ionensensitiven Elektrode eine für voltametrische Messung geeignete Feststoffelektrode als Arbeitselektrode benutzt wird. Die voltametrische Arbeitselektrode wird dabei zweckmäßig in einer potentiostatischen Meßschaltung betrieben.

Ein für die Durchführung der voltametrischen Messung geeignetes Elektrodensystem besteht aus der voltametrischen Arbeitselektrode einer aus einem chemisch indifferenten Metall bestehenden Gegenelektrode und einer Bezugselektrode, mit deren Hilfe eine konstante Arbeitsspannung zwischen der Arbeitselektrode und der Meßflüssigkeit aufrechterhalten wird.

Die voltametrische Messung erschließt neue Anwendungen für das eingangs beschriebene Meßverfahren. So konnte z.B. die sicherheitstechnische Aufgabe gelöst werden, Raumluft hinsichtlich Spuren von Chlorcyan kontinuierlich zu überwachen und die Überschreitung von Grenzwerten zu melden. Ein weiterer Vorteil besteht darin, daß das Meßsignal, d.h. der Elektrodenstrom, linear von der Stoffkonzentration abhängig ist. Die lineare Abhängigkeit ist von Bedeutung, wenn z.B. das Meßsignal zu Regelzwecken benutzt wird. Dagegen ist bei ionensensitiven

Le A 19 351

Elektroden das Meßsignal eine Galvanispannung, die entsprechend der Nernst'schen Gleichung logarythmisch abhängig ist von der Ionenkonzentration des Gases in der Flüssigkeit.

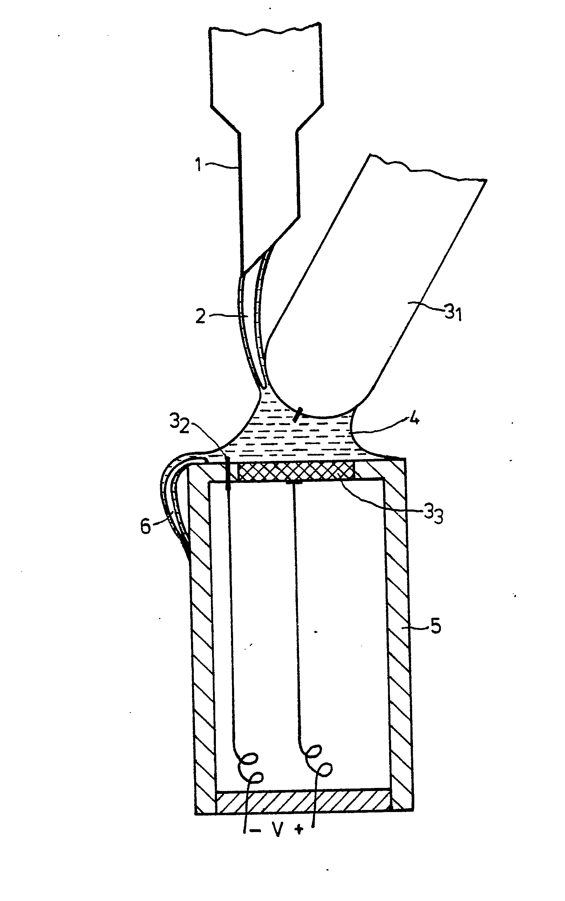

Im folgenden wird ein Ausführungsbeispiel der Erfindung anhand einer Zeichnung näher beschrieben. Die Figur zeigt die elektrochemische Meßzelle für eine voltametrische Messung.

Das Versprühen der Absorptionsflüssigkeit zusammen mit dem zu untersuchenden Gas erfolgt in der gleichen Weise wie in P27 23 310.9 beschrieben. Das Versprühen bewirkt bekanntlich eine intensive Durchmischung des Gases mit der Absorptionsflüssigkeit, so daß ein hoher Gasanteil in der Flüssigkeit absorbiert wird. Hierdurch wird eine optimale Meßempfindlichkeit gewährleistet. Auch die Abtrennung des nicht in der Flüssigkeit gelösten Gases wurde schon in P 27 23 310.9 beschrieben.

Anstelle der in P 27 23 310.9 beschriebenen ionensensitiven Elektrode wird hier jetzt ein Dreielektrodensystem zur voltametrischen Messung benutzt. Wie aus der Zeichnung ersichtlich, läuft die Absorptionsflüssigkeit kontinuierlich am unteren Ende einer Trennkammer 1 über einen hydrophilen Flüssigkeitsleiter, z.B. einen gekrümmten Glasstab 2, ab. Das Ende des Glasstabs 2 befindet sich innerhalb eines Dreielektrodensystems $3_1$, $3_2$, $3_3$, das unterhalb des Trennkammerausflusses angeordnet ist. Die Anordnung ist so dimensioniert, daß sich aufgrund der Adhäsionskräfte zwischen dem Ende des Glasstabs 2

Le A 19 351

und der voltametrischen Arbeitselektrode $3_3$ eine die Arbeitselektrode völlig bedeckende Flüssigkeitssäule 4 mit einer Höhe von 1 bis 2 mm ausbildet. Die Flüssigkeitssäule 4 steht außerdem mit der Bezugselektrode $3_1$ in Kontakt. Sie ist in gleicher Weise ausgebildet wie die in P 27 23 310.9 beschriebene Bezugselektrode für potentiometrische Messungen. Die Elektrode $3_3$ ist die voltametrische Arbeitselektrode. Sie besteht z.B. aus einem chemisch indifferenten Edelmetall oder Kohle. Außerdem sollte die zwischen Elektrode und Flüssigkeit stattfindende Reaktion nicht zu einer Feststoffablagerung auf der Elektrode führen. Die Gegenelektrode $3_2$ besteht ebenfalls aus einem Edelmetall, z.B. einem Platinstift, der in dem Schaft 5 der Arbeitselektrode $3_3$ fest verankert ist und ständig mit der Flüssigkeit in Berührung steht. Die voltametrische Messung beruht bei der potentiostatischen Arbeitsweise darauf, daß an die Elektrode $3_3$ eine Arbeitsspannung angelegt wird und der Stromfluß zur Gegenelektrode $3_2$ gemessen wird. Mit Hilfe der Bezugselektrode $3_1$ wird die Arbeitsspannung zwischen der Arbeitselektrode $3_3$ und der Meßflüssigkeit konstant gehalten. Eine detaillierte Beschreibung der potentiostatischen Arbeitsweise findet sich z.B. bei G. Kortüm, Lehrbuch der Elektrochemie, Chemie Verlag GmbH Weinheim, 2. Auflage 1950, Seite 396. Das beschriebene Dreielektrodensystem arbeitet ebenso wie die galvanische Meßzelle gemäß P 27 23 310.9 mit einem sehr geringen Flüssigkeitsvolumen. Hierdurch wird eine schnelle Ansprechzeit gewährleistet, da bei Konzentrationsänderungen des Meßgases ein schneller Austausch des Volumens stattfindet. Der Abfluß der Flüssigkeit aus der

0012415

voltametrischen Meßzelle erfolgt über einen weiteren
Flüssigkeitsleiter 6, dessen Ende am Schaft 5 der Arbeitselektrode anliegt.

## Patentansprüche

1) Verfahren zur Messung von Gasspuren, bei dem das zu untersuchende Gas zusammen mit einer Absorptionsflüssigkeit kontinuierlich zerstäubt wird, anschließend die flüssige Phase abgetrennt wird und in kontinuierlicher Strömung durch eine elektrochemische Meßzelle geleitet wird, dadurch gekennzeichnet, daß als Arbeitselektrode eine für voltametrische Messungen geeignete Feststoffelektrode verwendet wird.

2) Verfahren nach Anspruch 1, dadurch gekennzeichnet, daß die Arbeitselektrode in einer potentiostatischen Meßschaltung betrieben wird.

3) Elektrochemische Meßzelle zur Durchführung des Verfahrens gemäß Anspruch 1 und 2, gekennzeichnet durch ein Dreielektrodensystem, bestehend aus der voltametrischen Arbeitselektrode ($3_3$) aus einer chemisch indifferenten Gegenelektrode ($3_2$) und einer Bezugselektrode ($3_1$) mit deren Hilfe eine konstante Arbeitsspannung zwischen der Arbeitselektrode ($3_3$) und der Meßflüssigkeit (4) aufrechterhalten wird.

Le A 19 351

0012415

**Europäisches Patentamt**

**EUROPÄISCHER RECHERCHENBERICHT**

| Kategorie | Kennzeichnung des Dokuments mit Angabe, soweit erforderlich, der maßgeblichen Teile | betrifft Anspruch |
|---|---|---|
| A | DE - A - 2 002 808 (ZELLWEGER AG) <br><br> + Seite 3, Absatz 1, Zeilen 5-14, Absatz 2, Zeilen 1-3; Seite 17, Figur 1 + <br><br> -- | 1 |
| A | CH - A - 512 742 (VYZKUMMY USTAV ORGANICKYCH SYNTEZ) <br><br> + Gesamt + <br><br> -- | |
| A | DE - A - 2 360 294 (LABOR MÜS-ZERIPARI MÜVEK) <br><br> + Seite 5, Absatz 2, Zeilen 1-14 + <br><br> -- | 1 |
| A | FR - A1 - 2 347 681 (GEORGE KENT) <br><br> + Seite 10, Figur 1 + <br><br> -- | |
| A,D | DE - A1 - 2 723 310 (BAYER AG) <br><br> + Seite 1, Ansprüche 1-3; Seite 2, Ansprüche 5-9; Seite 13, Figur 2 + <br><br> -- | 1-3 |
| A | JOURNAL OF PHYSICS I.: SCIENTIFIC INSTRUMENTS, Vol.11,1978, <br><br> Bristol Instiut of Physics, Bristol, <br><br> R.GREEF: Instruments for use in electrode process research, Seiten1-12 <br><br> + Seiten 1,2 + <br><br> ---- | 1-3 |

## EINSCHLÄGIGE DOKUMENTE

KLASSIFIKATION DER ANMELDUNG (int.Cl.X) 3

G 01 N 27/46
// G 01 N 31/00

RECHERCHIERTE SACHGEBIETE (Int. Cl.X 3

G 01 N 27/00
G 01 N 31/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X   Der vorliegende Recherchenbericht wurde für alle Patentansprüche erstellt.

| Recherchenort | Abschlußdatum der Recherche | Prüfer |
|---|---|---|
| WIEN | 21-02-1980 | ERBER |